# EUROPEAN PATENT SPECIFICATION

(11) **EP 3 247 179 B1**
(45) Date of publication and mention of the grant of the patent: **30.12.2020**
(21) Application number: 15877393.7
(22) Date of filing: 12.01.2015
(51) Int. Cl.: H05H 1/46, A61N 1/44

(54) **PLUGGABLE PLASMA DISCHARGE TUBE DEVICE**
STECKBARE PLASMAENTLADUNGSRÖHRENVORRICHTUNG
DISPOSITIF DE TUBE À DÉCHARGE DE PLASMA ENFICHABLE

(43) Date of publication of application: 22.11.2017
(73) Proprietor: Wang, Shouguo, Beijing 102208 (CN)
(72) Inventor: Wang, Shouguo, Beijing 102208 (CN)
(74) Representative: Dargiewicz, Joanna
(86) International application number: PCT/CN2015/000023
(87) International publication number: WO 2016/112473

(56) References cited:
- WO-A2-2012/158443
- CN-A- 102 523 674
- CN-A- 102 523 674
- CN-A- 103 418 085
- CN-Y- 2 604 846
- CN-Y- 2 604 847
- DE-A1-102012 103 362
- GB-A- 2 501 484
- JP-A- 2008 041 495
- US-A1- 2013 153 545

## Description

### TECHNICAL FIELD

This invention involves a plasma device with a replaceable discharge tube. Specifically it is about a plasma discharge tube, which is connected (plugged in) to a hand-held shell. It uses a metal electrode to generate quasi-glow cold plasma, which can be used for disinfection and sterilization on sensitive surfaces, facial skin rejuvenation, treatment of skin tissue infections and destruction of cancer cells.

### BACKGROUND

It is generally proven that low temperature cold plasma can rapidly kill germs, viruses and cancer cells. The cold plasma can also be used for facial skin health as well, since the cold plasma contains large quantities of energetic materials such as electrons, ions, and active radicals, which can destroy various harmful bacteria.

The plasma used to kill germs and viruses on the skin requires a sustainable temperature, and in addition, the plasma is required to be glow discharge or quasi-glow, so as not to burn the skin.

Therefore, it is necessary to have low-temperature, cold plasma when applying to the skin. Its discharge intensity should be controllable and uniform, and have a quasi-glow discharge.

Traditionally, the normal capacitive coupling discharge used two electrodes to produce cold plasma between two electrodes. This plasma could be carried out to forma plasma beam by using a gas flow; example patent [public patent ZL200820180894.7], describes this atmosphere pressure plasma stream device. It is a typical dielectric barrier discharge using a capacitive coupling discharge method. A high voltage electrode is inside of an insulation tube, and a ground electrode is outside of the tube. The direction of plasma stream is perpendicular to the direction of the ionized field between the two electrodes. This type of discharge can easily create arcs between the inner high voltage electrode and the skin, while the skin is near to the tube nozzle. Such kind of plasma requires a certain gap to avoid arcs between the skin and tube nozzle. Therefore, it has relatively low energy efficiency. In addition, the capacitive coupling discharge device contains relatively complex manufacturing processes.

Another known method to produce cold plasma is inductive coupling discharge, that uses a solenoid structure with two electrodes connected separately to two output terminals of a power supply. This device [patent KR101260893] has its plasma generated from a spiral tube with coil electrodes. The electrodes are wrapped around the plasma generating tube through the inductively coupled electric field. This kind of discharge cannot be hand-held, and the electrode manufacturing process is difficult.

In patent CN101848595A, the plasma was produced using a radio-frequency (RF) power supply. The device includes a metal RF electrode and a ground electrode. Plasma was produced using the inert gas as a working gas, such as helium. The skin cannot contact the RF electrode during the discharging process. In addition, RF discharges may produce a radiation field, which may bring electromagnetic interference to surrounding electric appliances.

In patent GB2508176A, the plasma generator tip is connected to a RF power supply. This device produces an RF point discharge by directly ionizing the air. This type of discharge cannot be used on the skin and can only be used for cleaning the air.

Currently no pluggable (replaceable tip) design is found in those cold plasma devices. When the plasma is discharged on skin, the part that touches the skin should be replaced for each new patient to avoid cross-contamination.

When interposition plasma is used to treat the body tissue, the gas jetted from the plasma nozzle should be extracted out of the body. That requires the design of the channel of gas extraction. Currently no design of concentric casings suction device has been found.

As seen in past plasma devices, radio frequency and high frequency electric knives are used to cut the skin tissue. They are arc discharge plasma. These kinds of plasma are strong enough, though they are not suitable for general skin infection or for killing fungi. This type of device is not suitable for stimulating the activation of tissue cells for the purpose of skin rejuvenation.

In past plasma generation devices, the AC power is usually used. The general power supply voltage is 220V or 110V, power supply is directly turned from AC to DC, forming pulse signal through the high frequency-driven switch tube to drive high voltage transformer to produce high frequency and high voltage output. Using these power supply will cause sense of fear while discharging on the human skin, even if short-circuit protection, overcurrent protection and overvoltage protection are applied. By far no devices of plasma power connection with the low voltage DC power adaptor or battery have been found yet, and no plasma devices with power supply from the USB connection with computer are found for the disinfection of skin surface.

CN102523674 discloses an example of a hand-held plasma device.

### CONTENTS OF INVENTION

In order to overcome the various shortcomings in the cold plasma found in the devices as described above, this invention provides a plasma generating device according to claim 1. Preferred embodiments are disclosed in the dependent claims. In this single-electrode discharge unit, the difficulties in insulation are eliminated compared to devices that have two electrodes, which form a capacitive coupling discharge. This plasma invention also features a simple design and lower production costs.

To avoid cross contamination that might occur when the plasma discharge unit contacts with the skin, this invention has an easy replaceable, plug-in design.

This invention is used for the purpose of disinfection and sterilization of human skin, treatment of skin tissue infections and to kill cancer cells.

In order to better ensure safety when the plasma device discharges on the human body, the plasma generator requires a low-voltage DC power supply. Specifically, it can produce a plasma discharge when connected to the output terminal of a 12V power adaptor, a 5V battery via a USB interface, or powered by a computer USB interface.

The purpose of this power supply design is to isolate it from the common and dangerous 110V/220V source. This plasma power guarantees the safe use of the plasma devices via the connection design of the DC low voltage isolation.

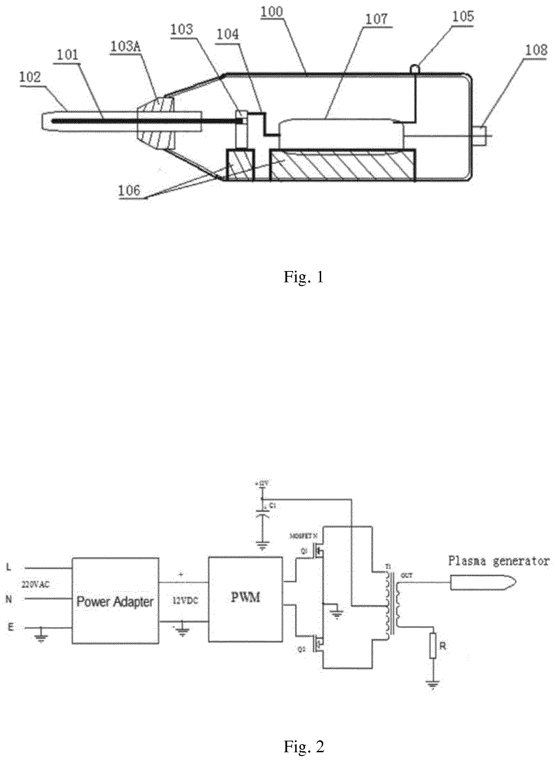

The power supply for this plasma device has a single-electrode output power. The other output terminal is connected to its own ground (as shown in Figure 2), or may be in suspension (is disconnected). Its output wattage can be controlled with a power control dial or adjusted with a remote digital switch.

The plasma power supply is a unipolar output source that has a voltage range of 4 to 25kV and a frequency range of 1 to 500kHz. The power output is 1 to 100W.

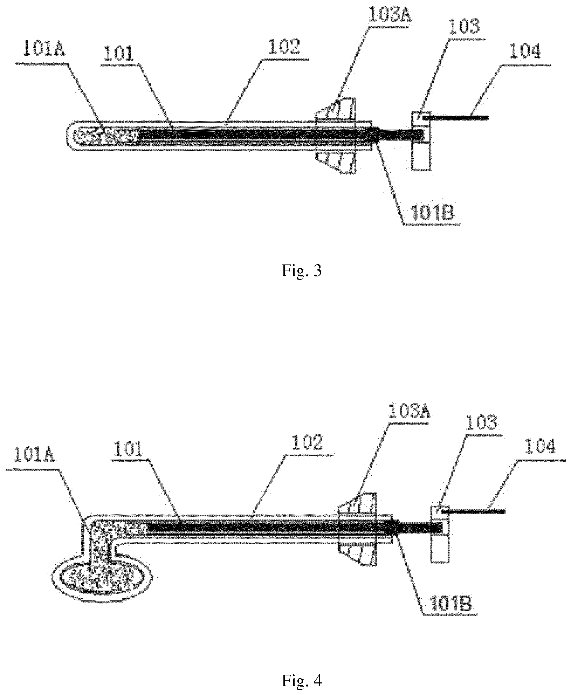

The discharge of the plasma discharge tube is as follows: a metal electrode is inserted into an insulating tube with a closed end, with a portion of the metal electrode exposed out of the insulating tube. The insulating material of the tube can be glass or ceramic. To accommodate the curved surface processing, the tip of the closed end of the plasma discharge tube may be in different shapes (FIG. 4).

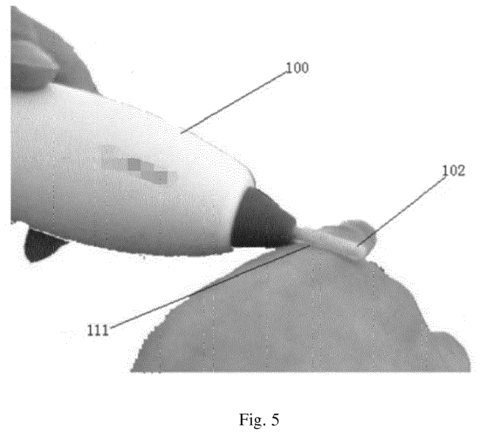

The discharge of the plasma discharge tube refers to: when the human body skin is close to the discharge tube within 2 mm or skin contacts the surface of the discharge tube, the space (air) between the skin and tube forms a quasi-polarized plasma charge breakdown (as shown in figure 5). If the distance between the skin and discharge tube exceeds 2 mm, plasma is no longer produced.

The plasma discharge tube features an exterior discharge mode. This tube is assembled as follows: a metal electrode is inserted into an insulating tube with a closed end, a portion of the metal electrode exposed outside of the tube. Between the metal electrode and the inner wall of the insulation tube is conductive powder; which may be aluminum, silver, or graphite. The opening of the insulating tube is sealed with sealing gum, which may be conductive silicone.

The plasma discharge tube is connected to the hand-held housing via plugging. The insulation tube and the hand-held shell are provided with the rubber location sleeve, and the insulation tube passes through the rubber location sleeve. The rubber location sleeve is arranged at one end of the hand-held shell, and one end of the metal electrode exposed outside of the insulation tube is inserted into the metal sleeve in the shell. The metal sleeve is fixedly arranged on the insulating fixed support in the shell, and is connected with one output end of the plasma power supply in the shell. And the closed end of the insulating tube is exposed outside of the hand-held shell.

The device features: a hand-held, insulated housing equipped with a power supply, a fixed support bracket, connecting wires, rubber material positioning sleeve and metal female hole jack. On the shell body is a power control dial and power input connectors. The advantage of this device is small, lightweight and easy to use inside the hand-held shell.

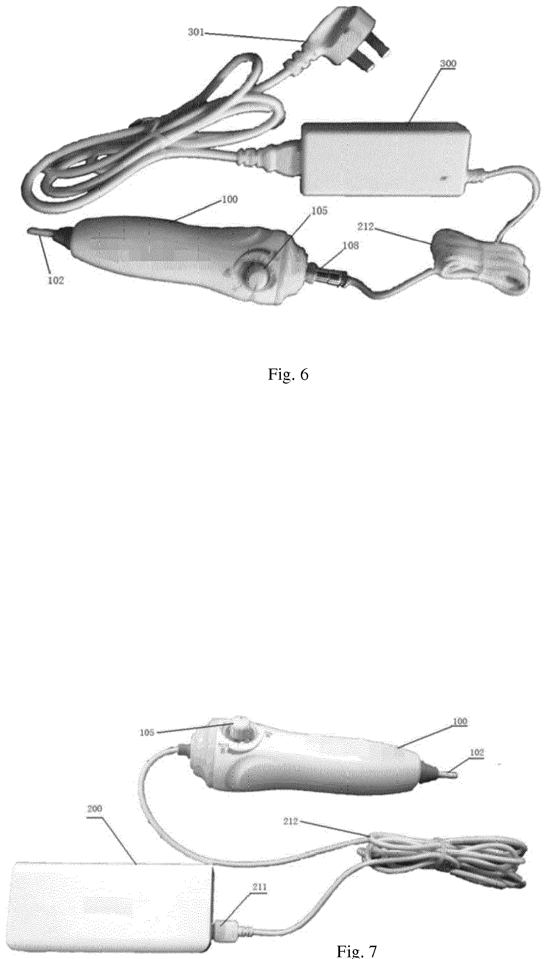

The device features an exterior discharge design. A connection plug of power input end is used to connect the power unit inside the shell body with the external power adapter that has a DC output voltage less than 12V (Figure 6), or connected to a 5V external battery via its USB interface (Figure 7).

The plasma discharge tube uses an inert gas supply featuring a medical insulation tube with both ends open. The insulation tube is connected to insulating connector (a stomatal opening) of the hand-held shell via a plugging. In the insulating connector (stomatal opening) there is an elastic seal. The insulation tube is inserted into the insulating connector (a stomatal opening) and connected through to intake channel of the shell body and to gas supply.

The plasma discharge tube with the gas supply described herein, features a metal electrode in the air-hole opening (stomatal opening) of the hand-held shell. The end of this electrode is placed inside of the air channel of the shell body and also can be wrapped by the insulating tube. This electrode is connected to an output of plasma power outside of the hand-held shell body through the electrode connector on the hand-held shell body. The other output of the power is connected with the ground wire of its circuit.

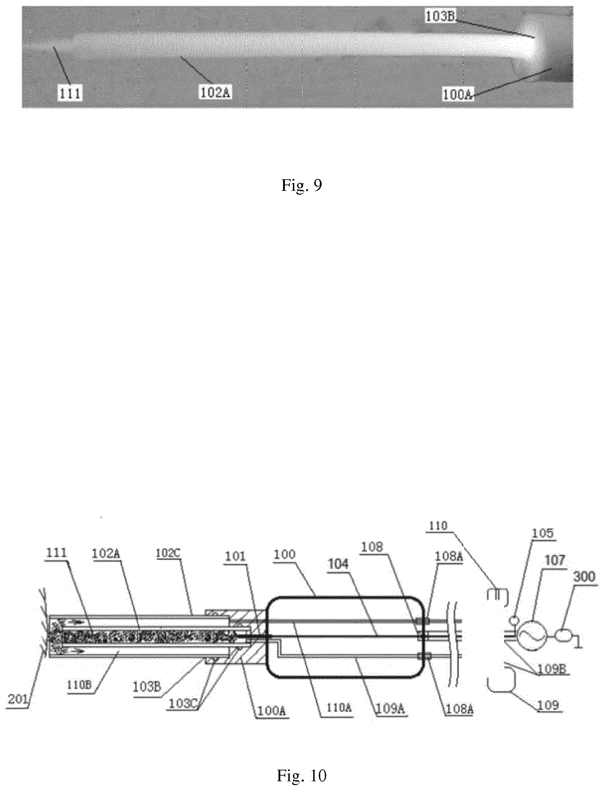

In the discharge of the plasma discharge tube with an inert gas used, the inert gas flows through the end of the electrode in the shell and is ionized to form a quasi-glow plasma jet stream. This plasma jet stream travels through the insulation tube and is sprayed out of the tube opening (as shown in Figure 9). The intensity of the plasma jet can be adjusted by the power wattage dial and by a gas volume switch.

When the discharge of the plasma discharge tube with an inert gas is used, there should be some space between the nozzle of the insulation tube and the electrode end. The insulation tube is required to be at least 60 mm in length. The length limit is set to avoid an arch discharge when the tube nozzle gets close to the skin.

When the discharge of the plasma discharge tube with an inert gas is used, another concentric outer insulation tube can be added outside of the insulation tube with some gap between the tubes and with outer tube 2 to 20 mm longer than the inner tube. The outer insulation tube is also plugged into the air-hole opening (stomatal opening) of the shell body and connects through to the intake tube in the shell and suction pump out of the shell. The gas sprayed out of the plasma discharge tube is then extracted by the suction pump from the intake channel between the inner and outer tube.

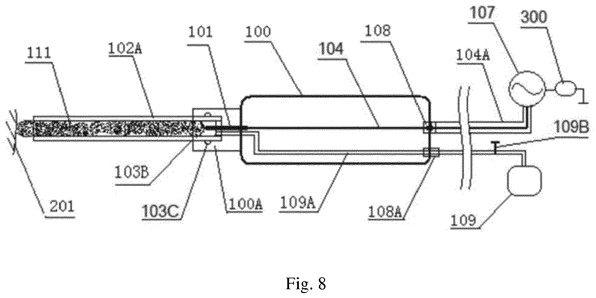

The inner discharge of the plasma discharge tube with an inert gas is used. The hand-held shell and the plasma power are connected via cable. The hand-held shell and the gas supply are connected via a gas tube (as shown in Figure 8).

Argon, helium or a mixture of both gases are used as gas supply.

### DIAGRAM DESCRIPTIONS

This present invention is further described in Figures 1-7. Figures 8-11 show examples not part of the invention.
Figure 1 shows a cross-sectional view of the tube outside discharge structure of the plasma discharge tube. (Implementation Example 1)
Figure 2 shows the working schematics of the technical implementation.
Figure 3 is a cross-sectional view of the plugging connection schematics in the plasma discharge tube. (Implementation Example 1)
Figure 4 shows an example of another type of end shape used in the plasma discharge tube. (Implementation Example 2)
Figure 5 is a photo of this present invention device, as it is being discharged on human skin.
Figure 6 is a photo of this present invention device with the attached power adapter. (Implementation Example 1)
Figure 7 is a photo of this present invention device with a USB interface connected to a battery pack. (Implementation Example 1)
Figure 8 is a cross-sectional view of inner discharge of plasma discharge tube with a gas supply. (Implementation Example 3)
Figure 9 is a photo of the plasma discharge inside tube with gas supply. (Implementation Example 3)
Figure 10 is a cross-sectional view of inner discharge structure of the plasma discharge tube having a gas and with another end shape. (Implementation Example 4)
Figure 11 is a cross-sectional view of the plasma discharge tube having a gas supply and a suction system. (Implementation Example 5)

### REFERENCE LIST OF THE PARTS IN ATTACHED DIAGRAMS

100 - hand-held insulating shell housing
100A - insulating connector with center hole, on the housing
101 - metal electrode
101A - conductive powder
101B - conductive silicon ring
102 - insulation tube (ceramic or glass)
102A - medical plastic tube
102B - medical plastic tube
102C - outer plastic sleeve
103 - metal positioning (location) sleeve
103A -rubber positioning (location) sleeve
103B -gas opening
103C - rubber sealing ring
104 - wire
104A - insulation wrapped wire
105 - power (wattage) control dial
106 - insulated positioning bracket
107 - plasma power supply
108 - electrode connection plug
108A - gas tube connector plug
109 - gas source
109A - intake tube
109B - gas volume control dial
110- suction pump
110A - exhaust tube
110B - space between two insulation tubes
111 - plasma
200 - battery
201 - skin
211 - USB connector
212 - twin connecting wire
300 - power adapter
301 - power plug

### SPECIFIC IMPLEMENTATION METHODS

### IMPLEMENTATION EXAMPLE 1

As shown in Figures 1, 2, 3 and 5 of this present invention, these are the details for Example 1 implementation. A hand-held insulating shell [100] which encloses a plasma power unit [107], insulated positioning bracket [106], metal positioning sleeve [103] and connecting wire [104] is shown. In the shell casing there are such components as a wattage adjustment control dial [105], and a power supply (electrode) connector plug [108]. The integrated components of this device allows for easy operation, and are lightweight and small in size.

As shown in Figures 1 and 3, the plasma discharge tube is connected via plugging to the hand-held shell through the positioning sleeve [103A]. The metal electrode [101] goes through the rubber positioning sleeve [103A], fastened on the shell [100], and is inserted into a metal sleeve [103] and connected to the metal sleeve. The metal sleeve [103], made of copper or stainless steel, is placed on an insulation bracket [106] of the shell [100] and connected to an output of power [107]. The closed end of the insulation tube [102] is exposed out of the hand-held shell [100]. The advantages of the plugging connection method between the plasma discharge tube and the shell [100] include precise positioning, convenient plugging, easy replacement after each use.

As shown in Figure 3, the plasma discharge tube is assembled as follows: A metal electrode is inserted inside an insulation tube [102] which is enclosed on one end. This metal electrode [101] can be made of copper, stainless steel, or tungsten copper alloy. Part of the metal electrode [101] is exposed outside of the insulation tube [102]. The gap between the metal electrode [101] and the inner wall of the insulation tube is filled with a conductive powder [101A], which may be aluminum, silver, or graphite. At the open end of the insulation tube [102] is a sealing ring [101B], which is made of conducting silicon. Fabricated this way, the plasma electrode tube does not have any air gaps and avoids unwanted discharge inside the tube.

As shown in Figure 5, the outside discharge of the plasma discharging tube: when the skin is within 2 mm from the discharge tube or contacts the surface of discharge tube (insulation tube) [102], the air space between the skin and tube is disrupted by a polarized charge on the tube [102] surface. This interaction produces a quasi-glow cold plasma discharge. If the discharge tube is more than 2 mm away from the skin, no plasma is produced. This discharge of the plasma occurs in the air, and no extra operating gas is needed. The intensity of the plasma discharge is regulated by the power control dial [105].

As shown in Figures 6 and 7, the transformer output of plasma power supply [107] of this plasma device requires an voltage range between 4 kV and 15 kV, and a frequency range between 1 kHz and 500 kHz, a wattage of the power supply between 0 W and 30 W. The other output of the power supply is connected to its own ground wire [Fig. 2].

The power source [107] of the plasma discharge device requires a low voltage DC power input, under 12V. For example, the power adapter [300] in (Figure 6) may be used, or a USB port [211] can be connected to a 5V battery [200] in (Figure 7). This power supply connection of plasma power supply [107] guarantees the safety when the plasma discharge is applied to the skin.

### Implementation Example 2

As shown in Figure 4, a bent head is used for the plasma discharge tube's end in Example 2 implementation, and that is the only differences between Example 2 implementation and that shown in Example 1. Compared with the shape of straight tube in Example 1, this discharge insulation tube with the closed end elbow-shaped produces plasma on the arched surface of its closed end. It is easy to observe the discharge from the side. All the other structures and functions of this present invention remain the same as Example 1 implementation.

In the embodiments of Example 1 and 2 implementations, one end of the plasma discharging tube is inserted into a metal positioning sleeve [103], which is connected via wire [104] to the output terminal of the power supply [107]. These connections are shown in Figures 1, 2, 3 and 4. Figure 2 shows a diagram of the connection of a single plasma electrode and its power supply. No other peripheral electrodes are needed with the plasma discharging tube.

### Implementation Example 3

As shown in Figure 8, the plasma discharge tube has a gas supply source and a internal discharge method. The device adopts a medical plastic tube [102A], with both end open, which is plugged into the connector [100A] of the hand-held shell [100]. This connector [100A] has a circular gas opening [103B] that enables a plug-in connection. Inside the circular gas opening [103B] is an elastic sealing ring [103C]. The medical plastic tube [102A] is plugged into the circular gas opening [103B] and connected through to the intake tube [109A] inside the hand-held shell [100]. This connection is used to form a channel through which gas may flow from the gas source [109].

As shown in Figure 8, there is a single metal electrode [101] inside the opening of the hand-held shell [100]. This metal electrode [101] can be made of copper, stainless steel, or Tungsten copper alloy. The top end of the metal electrode [101] is inside the gas channel of the plastic shell [100]. The exterior of the metal electrode can also be wrapped with a ceramic or quartz tube. This metal electrode [101] is connected via wire [104] to the electrode connector plug [108] that is attached to the hand-held plastic shell [100]. The electrode connector [108] is then connected by an insulated wire [104A] to an output terminal of a high voltage, high frequency power supply [107] that is outside of the shell [100]. The other output terminal of the power supply [107] is connected to a ground in its own circuit. The input end of the power supply [107] is connected to a power adapter [300], which provides 12V (or lower) DC.

As shown in Figures 8 and 9, when the inert gas from source [109] reaches the top end of the electrode [101], it is ionized to form a plasma stream (jet) [111], which travels through the medical insulation tube [102A] and sprays out of the tube tip. The strength of the plasma can be controlled by adjusting the output capacity of the power and the gas volume control dial [109B].

As shown in Figure 8, the nozzle of the insulation tube [102A] must be kept a minimum distance from the end of the electrode [101]. The length of the insulation tube [102A] shall be a minimum of 60 mm. This limit of length avoids an arc discharge of the electrode [101] direct on human body when human skin gets close to the nozzle of insulation tube.

As shown in Figure 8, the Example 3 implementation features an internal discharge of plasma discharge tube with an inert gas source. The hand-held shell [100] is connected to a power supply [107] via an insulated wrapped wire [104A], and is connected to the inert gas source [109] via the intake tube [109A].

The gas source [109] is inert gas which can be argon, helium or the mixture of the two.

The output of power supply [107] in the Example 3 implementation is required to have a voltage between 4 and 25 kV, a frequency of 1 to 500 kHz and power wattage of 1 to 100W. The other end of the power supply [107] is connected to its own ground as shown in Figure 2.

The power supply [107] required in the Example 3 implementation is a 12V (or lower) DC input. For example, the power supply [107] may be connected to a power adapter [300], which has an output voltage of 12V, as shown in Figure 6. This type of power supply [107] ensures the safety, when discharge is applied on the skin, via low voltage current isolation from the usual energy of a 220V or 110V power source.

### Implementation Example 4

As shown in Figure 10 is the schematic diagram of the structure of the plasma tube, in Example 4 implementation, in another end shape, discharging inside and with gas supply source. The difference from Example 3 implementation is the elbow-shaped end plastic tube [102B] used. In this case, plasma discharge may be applied are used in the treatment of oral skin infections.

### Implementation Example 5

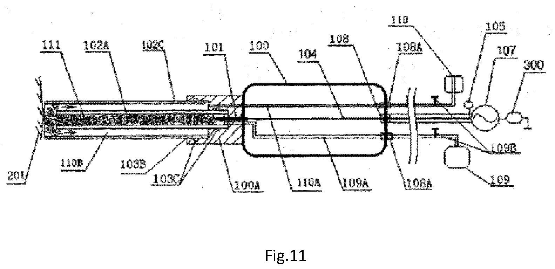

As shown in Figure 11, is a cross-sectional view of the Example 5 implementation that has an inert gas supply, a suction system and an internal discharge mode. In this diagram, an inner plastic tube [102A] has an outer concentric plastic sleeve [102C]. A space [110B] of 1 mm or less is kept between inner [102A] and outer plastic tube [102C]. The outer plastic sleeve [102C] is 2 to 20 mm longer than the inner plastic tube [102A]. The outer plastic sleeve [102C] is plugged into the plastic connector [100A] of the shell [100] and connects with the exhaust tube [110A] inside handheld shell [100] and suction pump [110] outside handheld shell [100],to form a continuous air/gas flow. The air sprayed from the end of the plastic discharge tube [102A] is then extracted out through the space [110B] between the outer plastic sleeve [102C] and the inner plastic tube [102A] and through the exhaust tube [110A] by the suction pump [110].

As shown in Figure 11 is Example 5 implementation. Its electrode structure, connections, gas supply and power supply requirements are exactly the same as in Example 3. The connecting inner plastic tube [102A] and the outer plastic sleeve [102C] are both made of medical plastic. The tube and sleeve can be a joint structure as long as there is a proper space between them.

As shown in Figure 11 is Example 5 implementation. The purpose of the suction system is for interventional plasma therapy on the human body. The plasma gas flowing out of the discharge tube end is then extracted out of human body through the suction pump [110] and the exhaust tube [110A].

It is necessary to point out that changes and modifications can be made to the above implementation examples by technical people in this area under the circumstance of not departing from the scope of the invention which is defined by the claims.

## Claims

1. A pluggable plasma discharge tube device for producing atmospheric pressure cold quasi-glow plasma, comprising:
a replaceable insulating discharge tube (102) and a hand-held shell (100) into which the replaceable insulating discharge tube (102) is inserted;
a power supply (107) provided inside the hand-held shell (100), the power supply (107) has an input voltage of 12V or less and comprises a transformer power unit having a high frequency, high voltage output terminal being the first output of the power supply (107) and the other output terminal being the other output of the power supply (107) connected to a ground wire, the transformer power unit input terminals being in connection with the power supply input voltage of 12V or less;
a single metal electrode (101) provided inside the replaceable insulating discharge tube (102); the single metal electrode (101) is connected with the first output of the power supply (107) by inserting into a metal sleeve (103) through a rubber positioning sleeve (103A); the metal sleeve (103) is fastened on an insulation bracket (106) of the hand-held shell (100) and electrically connected with the output of the power supply (107); a closed end of the replaceable discharge tube (102) is exposed out of the hand-held shell;
the gap between the metal electrode (101) and the inner wall of the replaceable insulating discharge tube (102) is filled with conductive powder (101A); and an open end of the replaceable discharge tube is sealed with a sealing ring (101B).

2. The pluggable plasma discharge tube device of claim 1, wherein the replaceable insulating discharge tube is made of ceramic, glass or plastic material.

3. The pluggable plasma discharge tube device of claim 1, wherein the power supply has an output frequency range between 1 to 500 kHz, a peak to peak voltage range from 4 to 25 kV, and an output wattage range from 1 to 100 W.

4. The pluggable plasma discharge tube device of claim 1 or 3, wherein the power supply (107) input voltage is provided by a battery (200); and a power output control dial (105) is provided on the hand-held shell (100).

## Patentansprüche

1. Eine steckbare Plasmaentladungsröhrenvorrichtung zum Erzeugen von kaltem Standard-Glühplasma bei Atmosphärendruck, umfassend:
Eine austauschbare isolierte Entladungsröhre (102) und ein tragbare Gehäuse (100), in das eine austauschbare isolierte Entladungsröhre (102) eingesetzt ist; eine Stromversorgung (107), die innerhalb des tragbare Gehäuses (100) angeordnet ist, Die Eingangsspannung der Stromversorgung (107) liegt unter 12 V und enthält ein Hochfrequenz-Transformator-Netzteil. Der Hochspannungs-Ausgangsanschluss ist der erste Ausgang der Stromversorgung (107) und der andere Ausgangsanschluss ist der andere Ausgang der Stromversorgung (107) mit dem Erdungskabel, dem Eingangsanschluss der Transformator-Leistungseinheit, die Eingangsspannung der Stromversorgung (107) liegt unter 12 V; die Metall-Einzelelektrode (101) wird in die austauschbare isolierte Entladungsröhre (102) eingestellt, die Metall-Einzelelektrode (101) wird durch die Gummipositionierungslochhülse (103A) in die Metallbuchse (103) eingeführt, somit mit dem ersten Ausgang der Stromversorgung (107) verbunden. Die Metallbuchse (103) ist am Isolierträger (106) des tragbaren Gehäuses (100) befestigt und elektrisch mit dem Ausgangsanschluss der Stromversorgung (107) verbunden. Das abgedichtete Ende des austauschbaren Entladungsrohrs (102) ist der Außenseite des tragbare Gehäuses (100) ausgesetzt; und der Spalt zwischen der Metallelektrode (101) und der Innenwand des austauschbaren isolierten Entladungsrohrs (102) ist mit leitfähigem Pulver (101A)gefüllt. Die Düse des austauschbaren Entladungsrohrs ist mit einem Dichtring (101B) abgedichtet.

2. Steckbare Plasmaentladungsröhrenvorrichtung nach Anspruch 1, wobei die austauschbare isolierte Entladungsröhre (102) eine Keramikröhre, eine Glasröhre oder eine Kunststoffröhre sein kann.

3. Steckbare Plasmaentladungsröhrenvorrichtung nach Anspruch 1, wobei der Ausgangsfrequenzbereich der Stromversorgung (107) 1 bis 500 kHz beträgt, der Spannungsspitzenbereich von Spitze zu Spitze 4 bis 25 kV beträgt und der Ausgang Der Leistungsbereich beträgt 1 ∼ 100W.

4. Steckbare Plasmaentladungsröhrenvorrichtung nach Anspruch 1 oder 3, wobei die Eingangsspannung der Stromversorgung (107) von einer Batterie (200) bereitgestellt wird; auf das tragbare Gehäuse (100) ist der Knopf (105) zur Steuerung des Leistungsausgangs versehen.

## Revendications

1. Un dispositif de tube à décharge plasma enfichable pour produire des plasma de quasi-lueur froide à pression atmosphérique, comprenant:
un tube à décharge isolant remplaçable (102) et une coque portative (100) dans laquelle le tube à décharge isolant remplaçable (102) est inséré; une alimentation électrique (107) disposée à l'intérieur de la coque portative (100), l'alimentation électrique (107) a une tension d'entrée de 12V ou moins, et comprend un bloc d'alimentation de transformateur ayant une fréquence élevée, la borne de sortie à haute tension étant la première sortie de l'alimentation électrique (107), et l'autre borne de sortie étant l'autre sortie de l'alimentation électrique (107) connectée à un fil de terre, les bornes d'entrée du bloc d'alimentation du transformateur et l'alimentation électrique (107) possèdent une tension d'entrée de 12V ou moins; une seule électrode métallique (101) est disposée à l'intérieur du tube à décharge isolant remplaçable (102); l'électrode métallique (101) est connectée à la première sortie de l'alimentation électrique (107) en insérant dans un manchon métallique (103) à travers un manchon de positionnement en caoutchouc (103A). Le manchon métallique (103) est fixé sur un support isolant (106) de la coque portative (100), et relié électriquement à la sortie de l'alimentation électrique (107). Une extrémité fermée du tube à décharge remplaçable (102) est exposée à l'extérieur de la coque portative (100); l'espace entre l'électrode métallique (101) et la paroi intérieure du tube à décharge isolant remplaçable (102) est rempli par poudre conductrice (101A). Une extrémité ouverte du tube à décharge remplaçable est scellé avec une bague d'étanchéité (101B).

2. Le dispositif de tube à décharge plasma enfichable selon la revendication 1, dans lequel le tube à décharge isolant remplaçable (102) est fait de céramique, de verre ou de matière plastique.

3. Le dispositif de tube à décharge plasma enfichable selon la revendication 1, dans lequel l'alimentation électrique (107) a une plage de fréquence de sortie entre 1 et 500 kHz, une plage de tension crête à crête de 4 à 25 kV, et une plage de puissance de sortie de 1 à 100 W.

4. Le dispositif de tube à décharge plasma enfichable selon la revendication 1 ou 3, dans lequel la tension d'entrée d'alimentation électrique (107) est fournie par une batterie (200); et un cadran de commande de puissance de sortie (105) est disposé sur la coque portative (100).
